# EUROPEAN PATENT SPECIFICATION

(11) **EP 4 004 237 B1**
(45) Date of publication and mention of the grant of the patent: **27.05.2026**
(21) Application number: 20744013.2
(22) Date of filing: 20.07.2020
(51) Int. Cl.: C12Q 1/6886, G16B 20/20, G16B 20/30, G16B 20/50, G16B 40/20

(54) **SYSTEMS AND METHODS FOR CELL OF ORIGIN DETERMINATION FROM VARIANT CALLING DATA**
SYSTEME UND VERFAHREN ZUR BESTIMMUNG EINER URSPRUNGSZELLE AUS VARIANT-CALLING-DATEN
SYSTÈMES ET PROCÉDÉS DE DÉTERMINATION DE CELLULE D'ORIGINE À PARTIR DE DONNÉES DE DE DÉTECTION DE VARIANT

(30) Priority: 22.07.2019 US 201962877238 P
(43) Date of publication of application: 01.06.2022
(60) Divisional application: 25218871.9
(73) Proprietor: F. Hoffmann-La Roche AG, 4070 Basel (CH); Roche Diagnostics GmbH, 68305 Mannheim (DE)
(72) Inventor: KURZ, David, Pleasanton, California 94588 (US); LIN, Hai, Pleasanton, California 94588 (US); LOVEJOY, Alexander, Pleasanton, California 94588 (US); LUONG, Khai, Pleasanton, California 94588 (US); TABARI, Ehsan, Pleasanton, California 94588 (US)
(74) Representative: Mewburn Ellis LLP
(86) International application number: PCT/EP2020/070472
(87) International publication number: WO 2021/013805

(56) References cited:
- WO-A1-2019/067092
- WO-A1-2020/132151
- WO-A2-2020/146554
- US-A1- 2013 195 843
- KEE PANG SOH ET AL: "Predicting cancer type from tumour DNA signatures", GENOME MEDICINE, vol. 9, no. 1, 1 December 2017 (2017-12-01), XP055682992, DOI: 10.1186/s13073-017-0493-2
- LYMPHOID NEOPLASIA ET AL: "Regular Article Genetic profiling of MYC and BCL2 in diffuse large B-cell lymphoma determines cell-of-origin-specific clinical impact", 28 March 2017 (2017-03-28), XP055738135, Retrieved from the Internet <URL:https://watermark.silverchair.com/blood747022.pdf?token=AQECAHi208BE49Ooan9kkhW_Ercy7Dm3ZL_9Cf3qfKAc485ysgAAA8QwggPABgkqhkiG9w0BBwagggOxMIIDrQIBADCCA6YGCSqGSIb3DQEHATAeBglghkgBZQMEAS4wEQQMVp49V6rl4jFF71i5AgEQgIIDd5wOp0YQ-NXjb7Km5Ir5TEKG8xeq1D5BZ-V84rpCXvTd8wvODWguSnd-mNrKMUREbCw6FJUuPAQyGOeKbWfOUkW3> [retrieved on 20201008], DOI: 10.1182/blood-2016-11-
- JORDINA ROVIRA ET AL: "MYD88 L265P Mutations, But No Other Variants, Identify a Subpopulation of DLBCL Patients of Activated B-cell Origin, Extranodal Involvement, and Poor Outcome", CLINICAL CANCER RESEARCH, vol. 22, no. 11, 20 January 2016 (2016-01-20), US, pages 2755 - 2764, XP055738136, ISSN: 1078-0432, DOI: 10.1158/1078-0432.CCR-15-1525

## Description

### FIELD

Embodiments of the invention related generally to classification of biological samples, and more specifically to cell of original classification.

### BACKGROUND

After naive B cells migrate from bone marrow to the germinal center, they undergo rapid proliferation and iterative rounds of somatic hypermutation, affinity maturation and clonal selection, as well as class switch recombination, with the aim of favoring the emergence of cells that produce antibodies with increased affinity for the antigen and capable of distinct effector functions. Pasqualucci, L. et al. (2018). Blood, 131, 2307-19. GCB lymphoma cells lack the expression of early post germinal center differentiation markers whereas ABC lymphomas display the transcriptional signature similar to that of activated B-cells. Kurtz D. et al. (2018). J Clinical Oncology, 36(28), 2845-53.

Diffuse large B cell lymphoma (DLBCL) shows a wide range of biological and molecular heterogeneity. Due to this diversity, personalized risk stratification and treatment are promising avenues to improving outcomes for DLBCL subjects, but these techniques rely on molecular or genomic data which are often hard to obtain in clinical settings. The most common classification scheme in DLBCL relies on determination of the tumor's cell of origin (COO). Two major COO classes, germinal center B cell-like (GCB) and activated B cell-like (ABC) lymphomas, have been defined, where patients with ABC subtype have inferior outcomes to standard therapy. See Alizadeh, A. et al. (2000). Nature, 403(6769), 503-511. Moreover, the ABC-like DLBCL has been shown to have superior response rates to targeted therapies including BTK inhibition. Unfortunately, current methods to assess COO remain limited by the need for tissue samples. Scherer, F. et al. (2016). Science Translational Medicine, 8(364). In addition, many methods to assess COO are dependent on expression data, based on RNA. Because most tissue samples are stored formalin fixed and paraffin embedded (FFPET), isolation of useable RNA from these samples is a challenge.

Accordingly, it would be desirable to provide new systems and methods for COO classification that can be performed on blood or plasma samples.

WO 2019/067092 A1 describes methods and materials for identifying a subject as having cancer in which the presence of members of two or more classes of biomarkers are detected, or two or more members of at least one class of biomarkers and the presence of aneuploidy are detected. US 2013/195843 A1 describes a method of identifying a subject as having B-cell non-Hodgkin lymphoma such as testing a sample from a subject for a mutation in one or more biomarkers. WO 2020/146554 A2 describes the use of molecular profiling data to identify biomarker signatures that predict a tumor primary lineage or organ group. WO 2020/028563 A1 describes methods of determining cell of origin for diffuse large B cell lymphoma using DNA for the analysis.

Daisuke Ennishi et al. ("Genetic profiling of MYC and BCL2 in diffuse large B-cell lymphoma determines cell-of-origin-specific clinical impact", Blood, The American Society of Hematology, vol. 129, no. 20, 28 March 2017, pages 2760-2770) describes that the incidence of *MYC*/*BCL2* genetic alterations and their clinical significance were largely dependent on COO subtypes. Jordina Rovira et al. ("MYD88 L265P Mutations, But No Other Variants, Identify a Subpopulation of DLBCL Patients of Activated B-Cell Origin, Extranodal Involvement, and Poor Outcome", Clinical Cancer Research, vol. 22, no. 11, 20 January 2016, pages 2755-2764) describes that MYD88 L265P mutations, but no other variants, identify a subgroup of DLBCL mainly of ABC origin, with extranodal involvement and poor outcome. Bolen Christopher et al. ("Systematic Analysis of the Prognostic Impact of Somatic Mutations in Diffuse Large B-Cell Lymphoma (DLBCL) with Evaluation of Cell-of-Origin Dependence: Results from the Phase 3 GOYA Trial in Previously Untreated DLBCL", Blood, The American Society of Hematology, vol. 130, no. Suppl. 1, 7 December 2017, page 2729) describes the characterization of the mutational profile of DLBCL by targeted next-generation sequencing, and evaluation of the prognostic impact of somatic mutations and its relation to COO. Jun Chin Ang et al. ("Supervised, Unsupervised, and Semi-Supervised Feature Selection", IEEE/ACM Transactions on Computational Biology and Bioinformatics, IEEE Service Center, New York, NY, US, vol. 13, no. 5, 1 September 2016, pages 971-989) describes the basic taxonomy of feature selection, and also reviews the state-of-the-art gene selection methods by grouping the literatures into three categories: supervised, unsupervised, and semi-supervised.

### SUMMARY OF THE DISCLOSURE

The claimed invention is defined by the appended claims.

The present invention relates generally to classification of biological samples, and more specifically to cell of original classification. In particular, embodiments of the invention relate to diffuse large B cell lymphoma cell of origin classification.

In a first aspect, a computer-implemented method for cell of origin determination for a type of cancer is provided. The method includes constructing a plurality of decision trees based on a collection of features, each decision tree comprising a random subset of features from the collection of features, wherein the features are genes that were identified based on one or more criteria from a plurality of blood or plasma samples from subjects having the type of cancer; and training the plurality of decision trees based on the collection of features to create a cell of origin classifier.

The type of cancer is diffuse large B cell lymphoma.

At least 10 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

In some embodiments, at least 20 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A,PRDM1, IRF4, KLHL14, PIM1, IGLL5, CDKN2A, TBL1XR1, ZEB2, CD79B, MYD88.

In some embodiments, at least 30 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A,PRDM1, IRF4, KLHL14, PIM1, IGLL5, CDKN2A, TBL1XR1, ZEB2, CD79B, MYD88.

The criteria for identifying a gene includes at least one of the following conditions: (1) at least about 2% of the subjects from a first cell of origin class or a second cell of origin class have a variant in the gene; (2) at most about 30% of all subjects with variants in the gene are unclassified; and (3) a ratio of a first cell of origin class to a second cell of original class GCB or a ratio of the second cell of original class to the first cell of origin class is at least 55:45.

The method further includes receiving a file comprising variant data from a patient, wherein the variant data was obtained from a blood or plasma sample from the patient; and using the cell of origin classifier to determine a cell of origin based on the variant data in the file.

In some embodiments, one of the features in the collection is variant location data.

In some embodiments, the variant location data includes at least one of: (1) a position of a variant in a gene; (2) information about a domain region of a protein which is modified in a variant; (3) information about a structural motif of a protein which is modified in a variant; and (4) functional region of a protein which is modified in a variant.

In some embodiments, one of the features in the collection is variant allele fraction data.

Described herein is a computer-implemented method for cell of origin classification for a type of cancer. The method includes constructing an ensemble model from a plurality of individual models, each individual model comprising an initial decision tree; and iteratively training each individual model to generate a plurality of successive decision trees that are added to each individual model, wherein each successive decision tree is configured to correct for an error in a previous decision tree, wherein each decision tree comprises a subset of features from a collection of features, wherein the features are genes that were identified based on one or more criteria from a plurality of blood or plasma samples from subjects having the type of cancer.

In some examples, each initial decision tree comprises a random subset of features from the collection of features.

In some examples, each individual model is trained for no more than 25 iterations.

In some examples, each individual model is trained for no more than 50 iterations.

In some examples, each individual model is trained for no more than 75 iterations.

In some examples, each individual model is trained for no more than 100 iterations.

In some examples, the type of cancer is diffuse large B cell lymphoma lymphoma.

In some examples, the collection of features includes at least ten features that are selected from the group of features consisting of EZH2_SNV, GNA13_SNV, BCL2 _Fusion, CD79B_SNV, PIM1_SNV, IGLL5_Indel, PIM1_Indel, SGK1_SNV, MYD88L273P_SNV, STAT6_SNV, TNFRSF14_SNV, P2RY8_SNV, CIITA_Indel, EGR1_SNV, ATG5_Indel, IRF4_SNV, S1PR2_SNV, SOCS1_SNV, CD58_Indel, and CNTNAP2_SNV.

In some examples, the collection of features includes at least 20 features that are selected from the group of features consisting of EZH2_SNV, GNA13_SNV, BCL2 _Fusion, CD79B_SNV, PIM1_SNV, IGLL5_Indel, PIM1_Indel, SGK1_SNV, MYD88L273P_SNV, STAT6_SNV, TNFRSF14_SNV, P2RY8_SNV, CIITA_Indel, EGR1_SNV, ATG5_Indel, IRF4_SNV, S1PR2_SNV, SOCS1_SNV, CD58_Indel, and CNTNAP2_SNV.

In some examples, the collection of features is selected from a larger pool of potential features based on an improvement to the ensemble models prediction accuracy.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a is a block diagram illustrating an embodiment of a computer system configured to implement one or more aspects of the present invention.
FIG. 2 is a plot that illustrates the number of samples containing ABC or GCB variants in each gene.
FIG. 3 is a diagram that illustrates an embodiment of a random forest algorithm.
FIGS. 4, 5A, and 5B are diagrams that illustrate various aspects of an embodiment of a gradient boosted decision tree algorithm.
FIG. 6 illustrates is a plot that illustrates the number of subjects each feature was detected in and the difference between the subjects' subtypes.
FIG. 7 is a diagram that illustrates an embodiment of the ensemble model.
FIGS. 8-10 present results from the ensemble model.

### DETAILED DESCRIPTION

The invention described here is a system and method based on a machine learning algorithm for COO determination, which is based on the variants present in a plasma sample, a blood sample, or a tissue sample, for example. Because the invention is based on variant calling, it is able call COO in plasma samples, blood samples, or in tissue samples, including FFPET samples, meaning it can be used much more broadly than previous methods.

Previously, a method for COO calling based on variant determination was described by Scherer, F. et al. Science Translational Medicine, 8(364)(2016) ("Scherer"). Briefly, the method described in Scherer is a Naive Bayes Classifier, assigning relative probabilities for GCB or ABC for each of 31 genes. Then, for a given sample, the probabilities from the variant-containing genes in the sample are combined to give an overall probability of ABC or GCB for a given sample. While this proved to be a useful technique for COO determination, the technique was based only on data from one publication (Pasqualucci, L. et al. Analysis of the coding genome of diffuse large B-cell lymphoma, Nature Genetics, 43, 830 (2011)), with only 48 DLBCL samples for training.

Described herein are (1) modifications and improvements to the COO determination method described by Scherer as well as (2) a new method of COO classification as described herein. The updates to the Scherer method are as follows:
1. New Machine Learning algorithms based on a broader set of publicly available data, using 40 genes for ABC vs GCB classifications
2. Use of further variant location data in the classifications-instead of simply using the gene which contained the variant, the domain or position within the gene can be incorporated
3. For plasma samples, use of the variant allele fraction (AF) as more information to weigh the probabilities of each variant-containing gene or location.

### Exemplary Computer System for Implementing a Machine Learning Algorithm

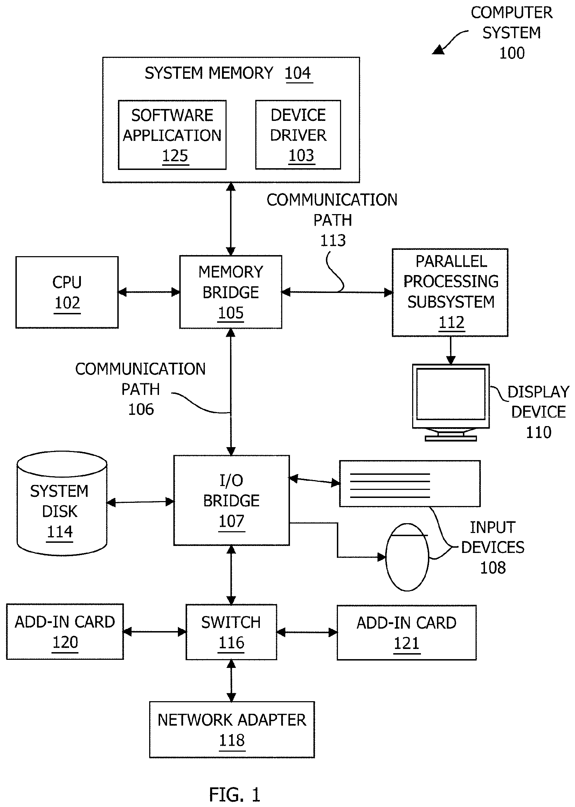

The machine learning algorithms can be implemented on a computer system. For example, FIG. 1 is a block diagram illustrating one embodiment of a computer system **100** configured to implement one or more aspects of the present invention. As shown, computer system **100** includes, without limitation, a central processing unit (CPU) **102** and a system memory **104** coupled to a parallel processing subsystem **112** via a memory bridge **105** and a communication path **113.** Memory bridge **105** is further coupled to an I/O (input/output) bridge **107**via a communication path **106,** and I/O bridge **107** is, in turn, coupled to a switch **116.**

In operation, I/O bridge **107** is configured to receive user input information from input devices **108** (e.g., a keyboard, a mouse, a video/image capture device, etc.) and forward the input information to CPU **102** for processing via communication path **106** and memory bridge **105.** In some embodiments, the input information is a live feed from a camera/image capture device or video data stored on a digital storage media on which object detection operations execute. Switch **116** is configured to provide connections between I/O bridge **107** and other components of the computer system **100,** such as a network adapter **118** and various add-in cards **120** and **121.**

As also shown, I/O bridge **107** is coupled to a system disk **114** that may be configured to store content and applications and data for use by CPU **102** and parallel processing subsystem **112.** As a general matter, system disk **114** provides non-volatile storage for applications and data and may include fixed or removable hard disk drives, flash memory devices, and CD-ROM (compact disc read-only-memory), DVD-ROM (digital versatile disc-ROM), Blu-ray, HD-DVD (high definition DVD), or other magnetic, optical, or solid state storage devices. Finally, although not explicitly shown, other components, such as universal serial bus or other port connections, compact disc drives, digital versatile disc drives, film recording devices, and the like, may be connected to I/O bridge **107** as well.

In various embodiments, memory bridge **105** may be a Northbridge chip, and I/O bridge **107** may be a Southbridge chip. In addition, communication paths **106** and **113,** as well as other communication paths within computer system **100,** may be implemented using any technically suitable protocols, including, without limitation, AGP (Accelerated Graphics Port), HyperTransport, or any other bus or point-to-point communication protocol known in the art.

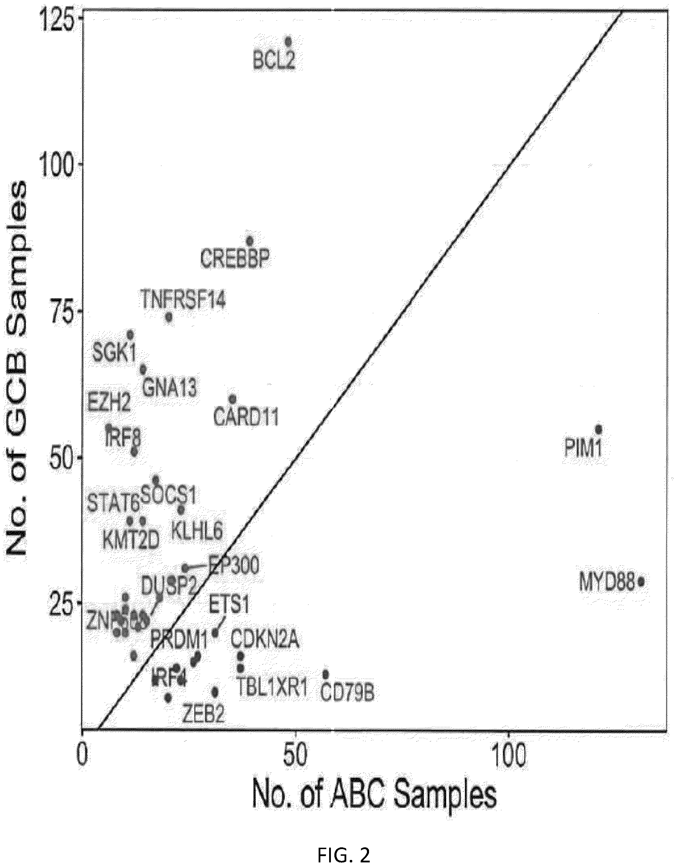

In some embodiments, parallel processing subsystem **112** comprises a graphics subsystem that delivers pixels to a display device **110** that may be any conventional cathode ray tube, liquid crystal display, light-emitting diode display, or the like. In such embodiments, the parallel processing subsystem **112** incorporates circuitry optimized for graphics and video processing, including, for example, video output circuitry. As described in greater detail below in FIG. 2, such circuitry may be incorporated across one or more parallel processing units (PPUs) included within parallel processing subsystem **112.** In other embodiments, the parallel processing subsystem **112** incorporates circuitry optimized for general purpose and/or compute processing. Again, such circuitry may be incorporated across one or more PPUs included within parallel processing subsystem **112** that are configured to perform such general purpose and/or compute operations. In yet other embodiments, the one or more PPUs included within parallel processing subsystem **112** may be configured to perform graphics processing, general purpose processing, and compute processing operations. System memory **104** includes at least one device driver **103**configured to manage the processing operations of the one or more PPUs within parallel processing subsystem **112.** The system memory **104** also includes a software application **125** that executes on the CPU **102** and may issue commands that control the operation of the PPUs.

In various embodiments, parallel processing subsystem **112** may be integrated with one or more other the other elements of FIG. 1 to form a single system. For example, parallel processing subsystem **112** may be integrated with CPU **102** and other connection circuitry on a single chip to form a system on chip (SoC).

It will be appreciated that the system shown herein is illustrative and that variations and modifications are possible. The connection topology, including the number and arrangement of bridges, the number of CPUs **102,** and the number of parallel processing subsystems **112,** may be modified as desired. For example, in some embodiments, system memory **104** could be connected to CPU **102** directly rather than through memory bridge **105,** and other devices would communicate with system memory **104** via memory bridge **105** and CPU **102.** In other alternative topologies, parallel processing subsystem **112** may be connected to I/O bridge **107** or directly to CPU **102,** rather than to memory bridge **105.** In still other embodiments, I/O bridge **107** and memory bridge **105** may be integrated into a single chip instead of existing as one or more discrete devices. Lastly, in certain embodiments, one or more components shown in FIG. 1 may not be present. For example, switch **116** could be eliminated, and network adapter **118** and add-in cards **120, 121** would connect directly to I/O bridge **107.**

### Sequencing

As described herein, variant calling data can be used in the systems and models describe herein for cell of origin classification. The variant calling data can be obtained in an electronic data file (i.e., a variant call format (VCF) file) that has been generated from sequencing nucleic acid molecules from a sample to generate sequencing data and performing primary and secondary analysis of the sequence data to identify variants. For example, the prepared nucleic acid molecules of interest (e.g., a sequencing library) can be sequenced using a sequencing assay as part of the procedure for determining sequencing reads for a plurality of microsatellite loci. Any of a number of sequencing technologies or sequencing assays can be utilized. The term "Next Generation Sequencing (NGS)" as used herein refers to sequencing methods that allow for massively parallel sequencing of clonally amplified molecules and of single nucleic acid molecules.

Non-limiting examples of sequence assays that are suitable for use with the methods disclosed herein include nanopore sequencing (US Pat. Publ. Nos. 2013/0244340, 2013/0264207, 2014/0134616, 2015/0119259 and 2015/0337366), Sanger sequencing, capillary array sequencing, thermal cycle sequencing (Sears et al., Biotechniques, 13:626-633 (1992)), solid-phase sequencing (Zimmerman et al., Methods Mol. Cell Biol., 3:39-42 (1992)), sequencing with mass spectrometry such as matrix-assisted laser desorption/ionization time-of-flight mass spectrometry (MALDI-TOF/MS; Fu et al., Nature Biotech., 16:381-384 (1998)), sequencing by hybridization (Drmanac et al., Nature Biotech., 16:54-58 (1998), and NGS methods, including but not limited to sequencing by synthesis (e.g., HiSeq^{™}, MiSeq^{™}, or Genome Analyzer, each available from Illumina), sequencing by ligation (e.g., SOLiD^{™}, Life Technologies), ion semiconductor sequencing (e.g., Ion Torrent^{™}, Life Technologies), and SMRT^{®} sequencing (e.g., Pacific Biosciences).

Commercially available sequencing technologies include: sequencing-by-hybridization platforms from Affymetrix Inc. (Sunnyvale, Calif.), sequencing-by-synthesis platforms from Illumina/Solexa (San Diego, Calif.) and Helicos Biosciences (Cambridge, Mass.), sequencing-by-ligation platform from Applied Biosystems (Foster City, Calif.). Other sequencing technologies include, but are not limited to, the Ion Torrent technology (ThermoFisher Scientific), and nanopore sequencing (Genia Technology from Roche Sequencing Solutions, Santa Clara, Cal.); and Oxford Nanopore Technologies (Oxford, United Kingdom).

### 1. MODIFIED AND IMPROVED MACHINE LEARNING ALGORITHM BASED ON A BROADER SET OF DATA, USING 40 GENES FOR ABC VS GCB CLASSIFICATIONS:

While the previous Scherer et al. COO caller was based only on 48 samples from Pasqualucci et al., described herein is an improved COO caller based on 945 samples from two different publications (Reddy, A. et al. (2017). Genetic and Functional Drivers of Diffuse Large B Cell Lymphoma. Cell, 171(2), 481-494; and Chapuy, B. et al. (2018). Molecular subtypes of diffuse large B cell lymphoma are associated with distinct pathogenic mechanisms and outcomes. Nature Medicine, 24(5), 679-690), allowing a broader basis for separation of ABC and GCB. Using data from these datasets, for each gene, the percent of DLBCL patients with a variant in the gene who were ABC vs GCB was determined. To choose genes to incorporate in the algorithm, genes were selected that met the following conditions: (1) Data from both datasets agreed on whether a variant in the gene was more likely to be present in GCB subjects or ABC subjects; (2) at least 25 subjects from one class (ABC or GCB) have variants in the gene; (3) at most 30% of all subjects with variants in the gene are unclassified; (4) the ratio of ABC:GCB or GCB:ABC subjects with a variant in the gene is at least 55:45. In other embodiments, data can be obtained from one or more public sources such as data from academic and/or medical journals, and/or from private sources such as privately run clinical trials and/or medical health records.

For example, genes that can be used as features in the ABC/GCB classifier algorithms described herein are listed in Table 1 with the proportion of samples labeled ABC, GCB or Unclassified (UNC) for each gene. These genes met the four conditions listed above. This table of genes is not meant to be exhaustive, and other genes that pass the criteria established above can also be included in the collection of features used by the classifier algorithm. The collection of features used by the classifier may include any combination of the genes listed in Table 1. For example, in some embodiments, the collection of features can include all 40 of the genes listed in Table 1. In other embodiments, the collection of features can include at least 5, 10, 15, 20, 25, 30, or 35 genes from Table 1. In other embodiments, the collection of features can include no more than 5, 10, 15, 20, 25, 30, 35, or 40 genes from Table 1.

**Table 1 proportion of samples labeled ABC, GCB or Unclassified for each gene**

| **Gene** | **ABC** | **GCB** | **UNC** | **Gene** | **ABC** | **GCB** | **UNC** |
|---|---|---|---|---|---|---|---|
| EZH2 | 0.09 | 0.85 | 0.06 | BCL6 | 0.29 | 0.48 | 0.23 |
| SGK1 | 0.11 | 0.70 | 0.19 | MYC | 0.31 | 0.50 | 0.19 |
| GNAI3 | 0.15 | 0.71 | 0.13 | PAX5 | 0.36 | 0.52 | 0.12 |
| IRF8 | 0.16 | 0.70 | 0.14 | ZNF608 | 0.38 | 0.54 | 0.08 |
| TNFRSF14 | 0.19 | 0.69 | 0.13 | DUSP2 | 0.38 | 0.52 | 0.11 |
| STAT6 | 0.18 | 0.65 | 0.17 | FOXO1 | 0.34 | 0.46 | 0.20 |
| BCL7A | 0.22 | 0.64 | 0.14 | EP300 | 0.40 | 0.52 | 0.08 |
| KMT2D | 0.23 | 0.63 | 0.15 | CCND3 | 0.44 | 0.31 | 0.26 |
| SOCS1 | 0.22 | 0.60 | 0.18 | ETS1 | 0.51 | 0.33 | 0.16 |
| RHOA | 0.26 | 0.67 | 0.08 | TMEM30A | 0.48 | 0.30 | 0.22 |
| BCL2 | 0.26 | 0.63 | 0.12 | PRDM1 | 0.59 | 0.35 | 0.07 |
| STAT3 | 0.24 | 0.61 | 0.15 | IRF4 | 0.46 | 0.27 | 0.27 |
| POU2F2 | 0.26 | 0.63 | 0.11 | KLHL14 | 0.53 | 0.28 | 0.19 |
| CD83 | 0.26 | 0.63 | 0.11 | PIM1 | 0.62 | 0.28 | 0.10 |
| NFKBIA | 0.27 | 0.65 | 0.08 | IGLL5 | 0.61 | 0.27 | 0.12 |
| CREBBP | 0.27 | 0.61 | 0.11 | CDKN2A | 0.57 | 0.25 | 0.18 |
| CD58 | 0.26 | 0.51 | 0.23 | TBL1XR1 | 0.62 | 0.23 | 0.15 |
| TET2 | 0.27 | 0.51 | 0.22 | ZEB2 | 0.62 | 0.20 | 0.18 |
| KLHL6 | 0.31 | 0.55 | 0.14 | CD79B | 0.75 | 0.17 | 0.08 |
| CARDI11 | 0.31 | 0.53 | 0.17 | MYD88 | 0.72 | 0.16 | 0.12 |

Examples of genes used in the ABC/GCB classifier are also shown in FIG. 2 with the number of subjects or samples containing ABC or GCB variants in each gene.

In other embodiments, condition (2) is set so that at least about 0.25, 0.5, 0.75, 1, 2, 3, 4, 5, 6, 7, 8, 9, or 10% of the subjects from one class (ABC or GCB) have variants in the gene. In other embodiments, condition (3) is set so that at most about 5, 10, 15, 20, 25, 30, 35, 40, 45, or 50% of all subjects with variants in the gene are unclassified. In other embodiments, condition (4) is set so that the ratio of ABC:GCB or GCB:ABC subjects with a variant in the gene is at least about 60:40, 65:35, 70:30, 75:25, 80:20, 85:15, or 90:10. In some embodiments, the genes selected for inclusion into the classification algorithm can be determined using any combination of the conditions listed herein.

Different machine learning algorithms may be useful for COO classification. For example, ensemble based machine learning algorithms and/or decision tree based machine learning algorithms can be used to perform COO classification. Ensemble methods combine multiple learning algorithm algorithms into a single aggregate model. For example, a model based on a plurality of decision trees can be considered an ensemble method. Decision tree algorithms include (1) a basic decision tree based on a plurality of conditions; (2) a bootstrap aggregating or bagging algorithm is an ensemble algorithm that combines predictions from a plurality of decision trees through a majority voting approach; (3) a random forest algorithm is a bagging-based algorithm where only a subset of features are selected at random for each decision tree to build a plurality (i.e., a forest) of decision trees; (4) a boosting algorithm builds each decision tree sequentially by minimizing the errors from previous models (i.e., decision trees) while increasing (i.e. boosting) the weight or influence of high-performing models (i.e. decision trees); (5) a gradient boosting algorithm uses a gradient descent algorithm to minimize errors in sequential model (i.e. boosting algorithm in (4)); and (6) optimized gradient boosting algorithm (i.e. XGBoost) which may employ parallel processing, tree-pruning, handling missing values, and regularization to reduce overfitting and/or bias in gradient boosting algorithms. Any of these algorithms, alone or in combination, can be used for the COO classifier.

### Random Forest Classifier

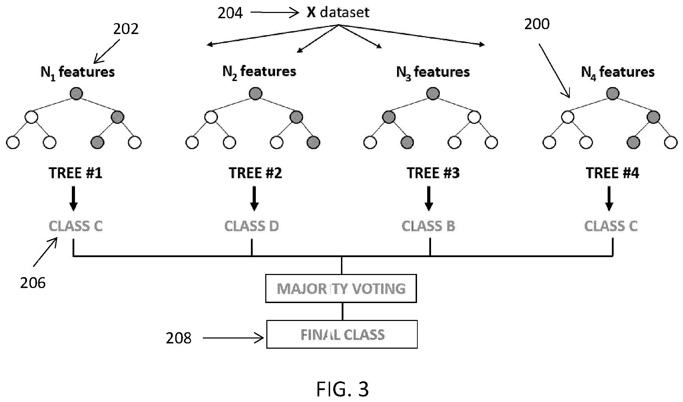

For example, a Random Forest classifier algorithm can be used as described herein to perform a COO classification, such as classifying a cell as ABC or GCB. FIG. 3 is a high level diagram that illustrates the general structure and process of a random forest classifier. In the random forest classifier, each decision tree 200 of a plurality of decision trees is constructed using a random subset of features 202 from of a collection of features 202, such as the genes listed in Table 1.

Once the plurality of trees 200 are constructed, the plurality of trees 200 can be trained using a training data set. For example, the training data set can be obtained through published papers in academic, medical, or scientific journals, such as Reddy and Chapuy as mentioned above. Alternatively or in addition, the training data set can be obtained from real world sequencing data and/or variant calling data obtained from patients.

After training, the random forest classifier can be used to classify sample data 204 (i.e. data contained in a variant call format (VCF) file) from a patient or subject. In other embodiments, the data can be obtained by sequencing the sample and then identifying the variants in the sequence data. Each tree 200 processes the sample data and independently determines and votes for a class 206 for the sample. The random forest classifier then determines the most popular class 208 using, for example, a majority or plurality voting process (i.e., class that receives the most votes is selected as the most popular class), which is assigned by the classifier to the sample.

Other parameters of the random forest classifier that can be adjusted include the number of trees and the number of features that are randomly selected at each node of the tree to determine the split. For example, the number of trees can be at least 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000. In some embodiments, the number of trees can be no more than 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000. In some embodiments, the number of features that are randomly selected at each node is at least 1, 2, 3, 4, 5, 6, 7, 8, 9, or 10. In some embodiments, the number of features that are randomly selected at each node is no more than 2, 3, 4, 5, 6, 7, 8, 9, or 10.

### (2). A NEW MODEL FOR COO CLASSIFICATION

### XGBoost Classifier

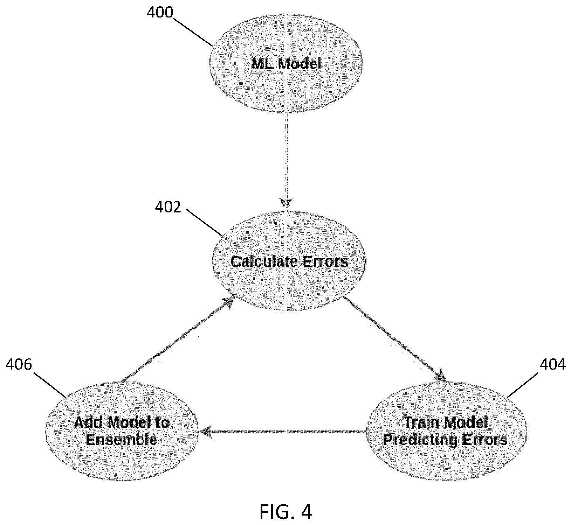

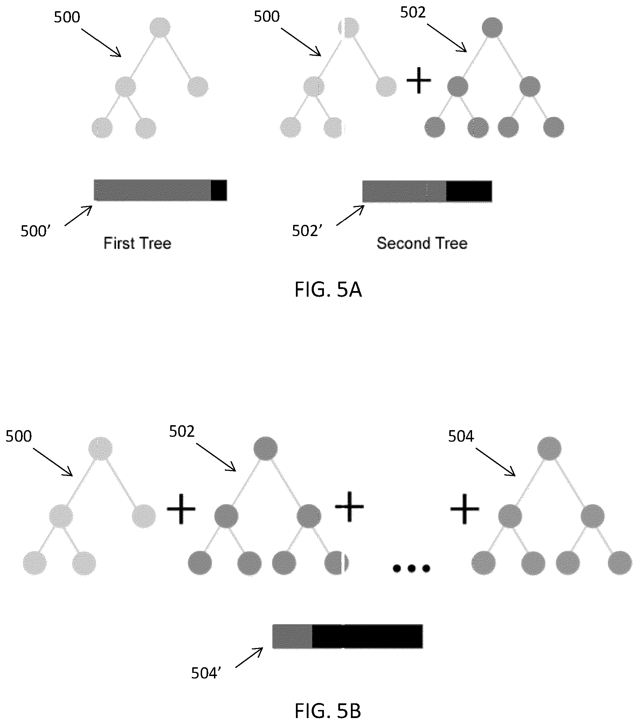

In another example, XGBoost can be used for the classifier. XGBoost is an optimized variation of a gradient boosted decision tree algorithm, and is shown at a high level in FIG. 4. One of the primary differences between the random forest algorithm and a boosted decision tree algorithm is in how the models are trained. In the random forest model, all the trees are trained together as a group, while in a boosted decision tree model, the training and construction of the model occurs iteratively, as shown in FIG. 4. For example, in a boosted decision tree algorithm, at step 400 a first decision tree can be constructed and trained. Then in step 402, the error of the first decision tree can be determined. Depending on the type of error function or algorithm used to determine and minimize the error, the model can be considered a boosted decision tree, a gradient boosted decision tree, or an optimized gradient boosted decision tree such as XGBoost. For example, a gradient boosted decision tree algorithm uses a gradient descent algorithm to minimize the error. In step 404, a second decision tree can be constructed using the feedback from the error determination step and then trained. In step 406, the new decision tree can be added to the first decision tree to form an ensemble model. Steps 402, 404, and 406 can be repeated until the ensemble model contains a selectable or predetermined number of trees, or the ensemble model satisfies one or more performance criteria, such as not exceeding a selectable or predetermined level of error. FIGS. 5A and 5B illustrate the iterative addition of decisions trees as described above in connection with FIG. 4, starting with a first tree 500, then adding a second tree 502, and then adding a N-th tree 504. Additionally, the error of the new decision tree is illustrated in the error bar 500', 502', 504' below the respective decision tree 500, 502, 504, with the ratio of the lighter portion to the darker portion of the bar representing the magnitude of the error.

To identify or determine the features for the model, targeted next generation sequencing (NGS) was performed on plasma samples from 310 and 43 previously untreated DLBCL subjects enrolled in the GOYA study (A Study of Obinutuzumab in Combination With CHOP Chemotherapy Versus Rituximab With CHOP in Participants With CD20-Positive Diffuse Large B-Cell Lymphoma (GOYA; https://clinicaltrials.gov/ct2/show/NCT01287741) and the Cavalli study (A Safety and Pharmacokinetics (PK) Study of Venetoclax in Participants With Non-Hodgkin's Lymphoma; https://clinicaltrials.gov/ct2/show/NCT02055820), respectively, using an adapted version of the AVENIO ctDNA Analysis kit (Roche Sequencing Solutions, Pleasanton, California) workflow with a custom DLBCL-specific panel. Cell free DNA (cfDNA) was isolated from plasma, up to 50ng of which was used as input for sequencing. Library preparation and NGS were done with a ctDNA analysis workflow and a custom panel of 314kb in size designed to cover regions relevant for cell of origin determination and minimal residual disease in DLBCL. Single nucleotide variants (SNVs), insertions and/or deletions (indels) and fusions were called from sequencing data using upgraded versions of the AVENIO ctDNA Analysis variant callers, and non-tumor specific variants were filtered out using publicly available data such as COSMIC, dbSNP and ExAC.

COO for these subjects were predetermined from tissue using NanoString Lymph2Cx, a gene expression based assay. Lymph2Cx assay reports a Linear Predictor Score (LPS) for each sample that determines its subtype. When the LPS score is close to the ABC/GCB threshold, the assay does not assign a subtype and reports UNCLASSIFIED. LPS was used as the ground truth and the target for the prediction.

In some embodiments, a threshold LPS score can be determined, and samples with a score below the threshold score by a set or predetermined amount, such as about 5, 10, 15, 20, or 25 % below the threshold LPS score (i.e., a GCB threshold score), can be identified as GCB, and samples with a score above the threshold LPS score by a set or predetermined amount, such as about 5, 10, 15, 20, or 25% above the threshold (i.e., a ABC threshold score), can be identified as ABC, and samples with a score between the GCB threshold score and the ABC threshold score can be left unclassified. For example, in one embodiment, the threshold LPS core can be about 2170, the GCB threshold score can be about 1910; the ABC threshold score can be about 2430, and scores between about 2170 and 2430 can be unclassified.

The dataset was divided into the following groups as summarized in Table 2 below.

**Table 2**

| **Group** | **Study** | **Total Subjects** | **ABC** | **GCB** | **Unclassified** |
|---|---|---|---|---|---|
| Building the model | GOYA | 230 | 99 | 109 | 22 |
| Validation set (blind during training) | GOYA | 80 | 21 | 44 | 15 |
| External dataset (blind set) | Cavalli | 41 | | 41 | |

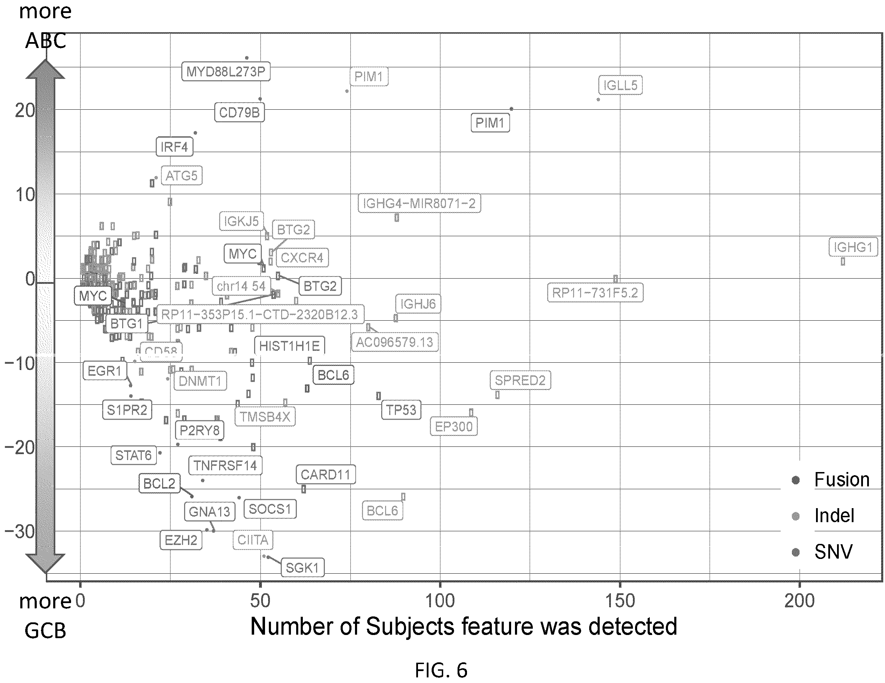

An ensemble of XGBoost models, which may also be referred to as XGBoost predictors when used for feature selection, with low subsampling rates on the 230 subjects in the training and test sets was used to select significant features. Features considered were variant type-gene pairs and/or genes with any indels or fusions or SNV (e.g. fusion in BCL2 or indel in PIM1). In one case (MYD88 L273P), only a single variant within a gene was considered, as it is a known common driver for ABC-type DLBCL. The models were trained against LPS and the top 20 most significant features from a much larger pool of potential features were selected based on the improvement of prediction accuracy (gain), as shown in Table 3 (list of potential features) and Table 4 (top 20 features selected from pool of potential features by ensemble of XGBoost models) below. The selected feature list includes 13 SNVs, 6 indels and one fusion. FIG. 6 shows the number of subjects each potential feature was detected in and the difference between the subjects' subtypes.

**Table 3: Potential features**

| Variants | | | |
|---|---|---|---|
| EZH2_SNV | HMCN1_SNV | CAPZA3_SNV | HIST1H2BC_Indel |
| GNA13_SNV | IRF8_SNV | CD58_SNV | B2M_Indel |
| BCL2_Fusion | IGHJ5_Indel | PTEN_Indel | CCND3_SNV |
| CD79B_SNV | AC096579.13_Indel | PIK3CD_Indel | TNFRSF14_Indel |
| PIM1_SNV | CDKN2A_Indel | TMSB4X_Indel | SRRM2_SNV |
| IGLL5_Indel | DKC1_Indel | MAP3K13_Indel | SPRED2_Indel |
| PIM1_Indel | ACTB_SNV | KLHL14_SNV | HIST1H2BC_SNV |
| SGK1_SNV | B2M_SNV | PCDH17_SNV | MYC_SNV |
| MYD88L273P_SNV | MALAT1_Indel | BORCS8-MEF2B_Indel | TBL1XR1_Indel |
| STAT6_SNV | XPO1_Indel | HIST1H4K_Indel | FOXO1_SNV |
| TNFRSF14_SNV | RP11-731F5.2_Indel | BCL6_SNV | IGHG1_SNV |
| P2RY8_SNV | HIST1H1E_SNV | HMCN1_Indel | HIST1H1C_SNV |
| CIITA_Indel | LINGO2_SNV | ZFP42_SNV | ITPKB_SNV |
| EGR1_SNV | BCL6_Indel | EPHA7_SNV | IGKJ5_Indel |
| ATG5_Indel | CREBBP_SNV | BCR_Indel | chr14 54_Indel |
| IRF4_SNV | IGHG4-MIR8071-2_Indel | WDPCP_SNV | CDKN2A_SNV |
| S1PR2_SNV | TLR2_SNV | DTX1_SNV | BTG2_SNV |
| SOCS1_SNV | STAT3_SNV | CD83_Indel | KLHL6_Indel |
| CD58_Indel | BCL6_Fusion | PRDM1_SNV | HIST1H3J_Indel |
| CNTNAP2_SNV | ZFP36L1_Indel | TP53_SNV | KLHL6_SNV |
| DNMT1_Indel | BCL2_Indel | IGHV2-70-IGHV3-71_Indel | HIST1H2BK_Indel |
| MC5R_SNV | IGHJ6_Indel | BCL10_SNV | ACTB_Indel |
| HIST1H2BO_SNV | GNA13_Indel | CXCR4_Indel | PIK3R1_SNV |
| CARD11_SNV | PAX5_Indel | BTG1_Indel | MYD88_SNV |
| CD83_SNV | BCL6-RP11-30015.1_Indel | RP11-353P15.1-CTD-2320B12.3_Indel | NFKBIA _SNV |
| PTEN_SNV | EP300_Indel | TNFAIP3_SNV | NOTCH2_SNV |
| IGHG1_Indel | BCL2_SNV | ACTG1_Indel | BTG1_SNV |
| IGHV2-70_Indel | PDCD1LG2_SNV | RP11-731F5.1_Indel | CREBBP_Indel |
| BRCA2_Indel | XBP1_Indel | BTG2_Indel | MSH6_SNV |
| TP53_Indel | CXCR5_SNV | BCL7A_Indel | PCSK5_SNV |
| MAGEC2_Indel | GK2_SNV | CXCR4_SNV | PSD3_Indel |
| EP300_SNV | PCDHA1_Indel | FAS_Indel | F2RL2_SNV |
| TET2_Indel | chr14 14_Indel | MUM1_Indel | NFATC1_Indel |
| SGK1_Indel | FAS_SNV | BMS1P8-ENPP7P13_Indel | PCDH17_Indel |
| PCLO_SNV | CDH19_SNV | ACTG1_SNV | MIR5195_Indel |
| MAGEC2_SNV | LRMP_Indel | NMRAL2P_Indel | PLCG2_SNV |
| CD70_SNV | MAP2K1_SNV | CACNA1S_SNV | KRAS_SNV |
| S1PR2_Indel | CD53_SNV | HIST1H2AC_Indel | KLF10_SNV |
| DNAH5_Indel | RHOH_Indel | HIST1H3C_Indel | ZFP42_Indel |
| HIST1H1D_SNV | NFIA_SNV | TCL1A_Indel | BTG3_SNV |
| LRRN3_SNV | TNFAIP3_Indel | TBL1XR1_SNV | GSDMC_SNV |
| BNC2_SNV | TMSB4X_SNV | IGHA1_Indel | SCML4_SNV |
| ITPKB_Indel | NEXMIF_Indel | HIST1H3F_SNV | CSMD1_SNV |
| KMT2D_SNV | LPP_Indel | EGR1_Indel | NCOA3_Indel |
| MEX3C_SNV | IGHJ4_Indel | NOTCH1_SNV | CSMD1_Indel |
| TSPOAP1-AS1_Indel | IGHV4-59_Indel | THBD_SNV | IGHM_Indel |
| BCR_SNV | RFTN1_SNV | MC5R_Indel | FAT1_Indel |
| HIST1H1E_Indel | DUSP2_SNV | P2RY8_Indel | ZEB2_SNV |
| IMMP2L_Indel | DMD_Indel | HIST1H2BJ_Indel | ASAP1_Indel |
| BRINP3_SNV | chr14 15_Indel | CLSTN2_SNV | AHCYL1_Indel |
| BCL10_Indel | ERICH1_SNV | CD36_SNV | WRAP73_SNV |
| FGFR4_Indel | GSDMC_Indel | DNAH5_SNV | IGHV3-7_Indel |
| POU2F2_SNV | C1orf194_SNV | ID3_Indel | PSD3_SNV |
| HIST1H2AM_SNV | DHX33_Indel | CAPZA3_Indel | MYO15A_SNV |
| MYC_Indel | SORCS2_SNV | POM121L2_SNV | MPEG1_Indel |
| IGLC2_Indel | MIR4537_Indel | PDCD1_SNV | CFL1_SNV |
| BIRC3_Indel | chr14 9_Indel | AFF3_Indel | KLK13_Indel |
| IRF8_Indel | XBP1_SNV | MFHAS1_SNV | RHOA_Indel |
| PPP4C_SNV | HIST1H2AE_SNV | IGKC_SNV | FGFR4_SNV |
| MYC_Fusion | IGLC3_Indel | SRRM2_Indel | LIPH_Indel |
| STAMBPL1_Indel | HIST1H2AG_Indel | LAMA1_SNV | EZH2_Indel |
| AC092170.1-AC009312.1_Indel | DPAGT1_Indel | ACAD8_SNV | BCL11A_SNV |
| BRCA2_SNV | BCHE_SNV | TCL1A_SNV | HOOK2_Indel |
| RIMS2_SNV | CD70_Indel | BTK_Indel | YTHDF2_SNV |
| DSEL_SNV | STAT3_Indel | ZCCHC7-RP11-397D12.4_Indel | HIST1H4K _SNV |
| IRF1_SNV | ZEB2_Indel | HIST1H2BM_SNV | EDIL3_SNV |
| RASSF9_SNV | KCND2_SNV | IZUMO3_SNV | HIST1H3A_SNV |
| MEF2B_SNV | ADAMTS9_SNV | CACNA1E_Indel | LINC00470-RP11-476K15.1_Indel |
| HIST1H2AC_SNV | CARD11_Indel | AC246787.1_Indel | NCAPD3_SNV |
| JUNB_SNV | KMT2D_Indel | TNFRSF14- | PABPC1_SNV |
| | | AS1_Indel | |
| NFATC1_SNV | HIST1H2BO_Indel | Clorf194_Indel | CTNND2_SNV |
| COL22A1_Indel | ETS1_Indel | HIST1H3F_Indel | MORN1_Indel |
| KLHL14_Indel | LIPN_SNV | IQGAP2_Indel | IGKJ1_Indel |
| MYD88_Indel | MAGEB16_SNV | MIR5571_Indel | RUBCNL_Indel |
| BCL2-KDSR_Indel | NTNG1_SNV | HIST1H3A_Indel | TMEM30A_Indel |
| IGHA2_Indel | POU2AF1_Indel | IGKJ3_Indel | TNFRSF1A_SNV |
| KIF2B_SNV | IGHV1-18_Indel | KLHL4_SNV | IRF1_Indel |
| ELAVL1_SNV | EHBP1_Indel | FBXW7_SNV | PLCG2_Indel |
| FAT1_SNV | MAGEB16_Indel | CRISPLD1_SNV | ZNF577_SNV |
| SLITRK1_SNV | AC118562.1-RP11-427M20.1_Indel | LRP1B_SNV | HIST1H2BG_SNV |
| IGLV7-43_Indel | IGLV3-1_Indel | ARID1A_Indel | RBFA_Indel |
| CD274_Indel | HDAC7_SNV | IGHV3-23_Indel | MRO_SNV |
| MED12_Indel | IRF4_Indel | IGHJ2P_Indel | IGKV3D-11_Indel |
| ADAMTS9_Indel | HIST1H2AE_Indel | EIF2AK3_Indel | ELK2BP-IGHA2_Indel |
| FOXO1_Indel | MAP3K13_SNV | IGHM_SNV | IGLV1-41_Indel |
| DTX1_Indel | ADAMTS16_SNV | EPHA7_Indel | IGKV3-20_Indel |
| BACH2_Indel | IGHD2-2_Indel | HIST1H2BJ_SNV | PLCL1_Indel |
| EBF3_SNV | BRAF_Indel | REL_Indel | SMAD4_SNV |
| ZFP36L1_SNV | ST6GAL1_Indel | IGHG3_Indel | SLC38A9_Indel |
| IGHJ3P_Indel | BTK_SNV | CRYAB_Indel | CDH19_Indel |
| ATP8B1_SNV | HIST1H2BD_Indel | MDH1_SNV | HIST1H3D_Indel |
| CD274_SNV | KIAA2022_SNV | NFKBIA_Indel | IGHD3-3_Indel |
| PCDHA6_SNV | IZUMO3_Indel | IGF2BP2_Indel | LIPH_SNV |
| NEAT1_Indel | HAS2_SNV | LINC02202_Indel | RTN4_Indel |
| MFHAS1_Indel | IGHV4-34_Indel | GRM7_Indel | MPDZ_Indel |
| HIST1H1B_SNV | TMEM30A_SNV | ABCB11_Indel | PLEKHG1_Indel |
| RMI2_Indel | ADAMTS16_Indel | USP34_SNV | IGHG4_SNV |
| MPEG1_SNV | HIST1H2AL _SNV | DSEL_Indel | ELAC1_Indel |
| KLHL25_SNV | IGLJ2-IGLC2_Indel | chr14 11_Indel | IGHJ4_SNV |
| ARID1A_SNV | ACAD8_Indel | IGLJ3_Indel | ELAC1_SNV |
| ERICH1_Indel | KLK13_SNV | HIST1H3C_SNV | TAS2R16_SNV |
| HIST1H2AJ_Indel | HIST1H3H_SNV | MYO15A_Indel | MPDZ_SNV |
| REL_SNV | MYC-PVT1_Indel | EHBP1_SNV | HIST1H2BG_Indel |
| IGHG2_Indel | CASC11-MYC_Indel | CTNND2_Indel | IGLV3-9_Indel |
| IGKC_Indel | HIST1H2BN_Indel | TGFBI _SNV | PLCL1_SNV |
| HIST1H3D_SNV | chr14 17_Indel | RASSF9_Indel | KRAS_Indel |
| CRISPLD1_Indel | HIST1H1C_Indel | IGHG3_SNV | SERPINE3_SNV |
| IGKJ4_Indel | ID3_SNV | LIPM_SNV | ADAMTS1_SNV |
| CTNNA2_SNV | NOTCH1_Indel | DLAT_Indel | RHOA_SNV |
| CCND3_Indel | CXCR5_Indel | CDH12_SNV | chr2 28_Indel |
| PXDN_SNV | HIST1H3I _SNV | IGHG2_SNV | EPS8_Indel |
| ZNF608_SNV | ABCB11_SNV | ELAVL1_Indel | DRG2_SNV |
| BCHE_Indel | HIST1H2AG_SNV | KHDRBS3_SNV | ATP8B1_Indel |
| CSMD3_SNV | BCL11A_Indel | CSMD3_Indel | COL22A1_SNV |
| LRRTM4_SNV | COL24A1_SNV | AFF1_Indel | BRINP3_Indel |
| TET2_SNV | FAM30A_Indel | NOTCH2_Indel | HIST1H1B_Indel |
| KLHL4_Indel | CDKN2B_SNV | CLSTN2_Indel | CDKN2B-AS1_Indel |
| PIK3C2G_SNV | IGHA2-AL928742.1_Indel | IGHV1-69_Indel | CD79B_Indel |
| GRM7_SNV | HIST1H1D_Indel | ADAMTS1_Indel | ZNF608_Indel |
| HIST1H2BK_SNV | HIST1H2BD_SNV | TRPS1_SNV | HIST1H3B_SNV |
| IGLV7-43-IGLVI-42_Indel | XPO1_SNV | BNC2_Indel | SLC22A16_SNV |
| IGHV4-39_Indel | ACVR2A_Indel | IGHV3-48_Indel | chr22 29_Indel |
| SI_SNV | DUSP2_Indel | CNTNAP2_Indel | RP11-44H4.1_Indel |
| ATP1B4_Indel | ARHGAP5_Indel | NFRKB_SNV | RER1_Indel |
| IGHG4_Indel | | | |

**Table 4 Top 20 most significant features selected by the XGBoost models**

| **Feature** | **Gain total** |
|---|---|
| EZH2_SNV | 4.320095226 |
| GNA13_SNV | 3.354813644 |
| BCL2_Fusion | 3.06984999 |
| CD79B_SNV | 2.329705116 |
| PIM1_SNV | 2.262074135 |
| IGLL5_Indel | 1.906037699 |
| PIM1_Indel | 1.71874648 |
| SGK1_SNV | 1.569275931 |
| MYD88L273P_SNV | 1.538712245 |
| STAT6_SNV | 1.535833008 |
| TNFRSF14_SNV | 1.386520606 |
| P2RY8_SNV | 1.355164869 |
| CIITA_Indel | 1.311255188 |
| EGR1_SNV | 1.255471684 |
| ATG5_Indel | 1.246242948 |
| IRF4_SNV | 1.226126648 |
| S1PR2_SNV | 1.203884924 |
| SOCS1_SNV | 1.202961181 |
| CD58_Indel | 1.171491699 |
| CNTNAP2_SNV | 1.079906928 |

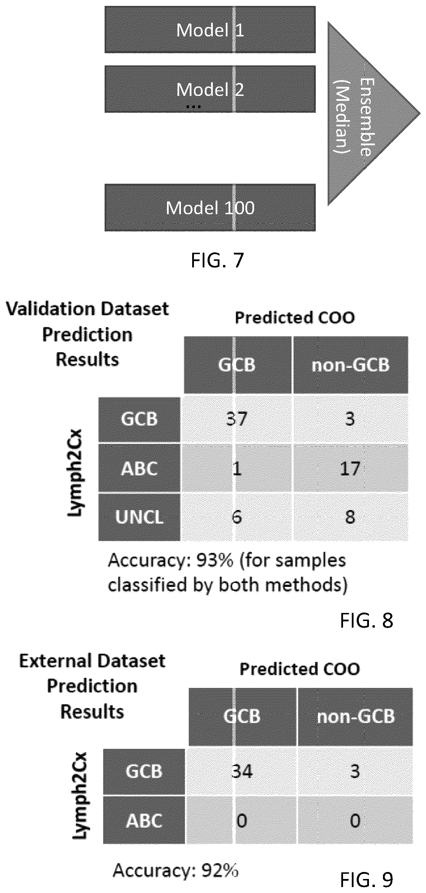

An ensemble model of 100 XGBoost models was built that were parameterized similarly to compensate for the high variability due to data sparsity. See FIG. 7. Only 25 iterations of training were used for prediction to avoid overfitting to the training data. The median of predicted score from these models was used. While the range of predicted scores for the ABC and GCB subtypes were differentiable, the score ranges for the subjects in the UNCLASSIFIED subtype overlapped the ones for ABC subjects. Hence, the reported subtypes were labeled GCB and non-GCB.

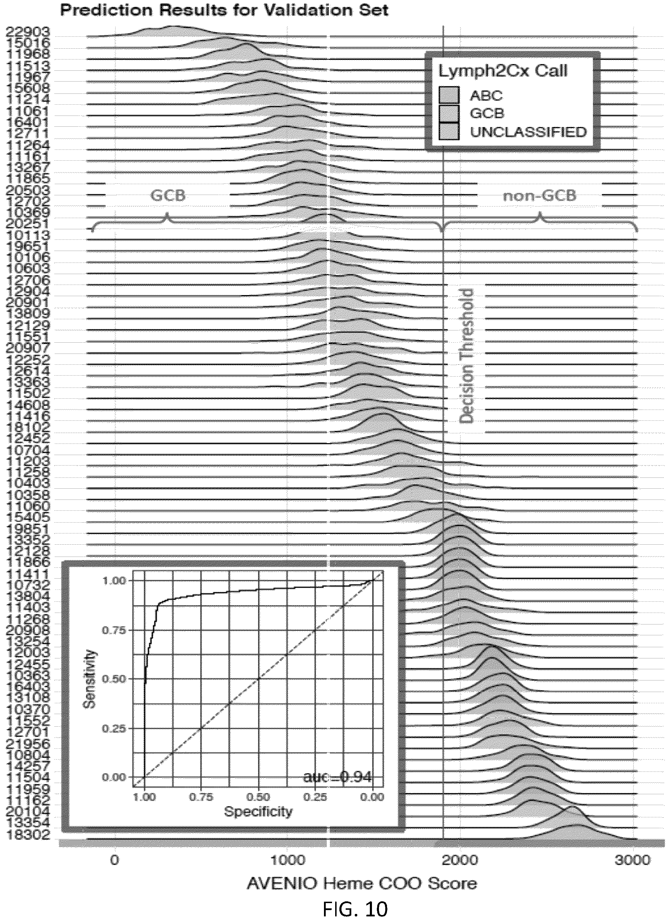

The ensemble model did not detect any relevant variants in eight and four of the subjects in the validation and external datasets, so no calls were returned for them. One to ten features were detected for the remaining subjects in the validation and external datasets, with 62% of the subjects having less than four features. The results are summarized in FIGS. 8-10.

The results show that variant calls from plasma can be effective as a tissue-free method for COO calling in DLBCL subjects. The ensemble model described herein is a significant improvement over the cfDNA-based COO classifier described by Scherer. An ensemble of boosting algorithms can be an effective strategy to compensate for the bias introduced be a highly sparse dataset.

### 2. Use of further variant location data in the classification algorithms:

The Scherer at al. method only uses the presence of a variant in a gene to contribute to COO classification. However, not every variant in a given gene is likely to have the same impact. For example, variants in kinase or other enzymatic regions of proteins are more likely to be oncogenic. For this reason, in some embodiment, incorporation of more information about the variants within a gene can be used to further refine COO calls. Using the exact position and/or information about the domain and/or structural motif and/or functional (i.e., enzymatic or binding) region of the protein which is modified within a gene can improve COO calling. Therefore, in some embodiments, the variant location data and/or the information regarding the modification of a particular domain and/or structural motif and/or functional region can be additional features that may be included in the collection of features analyzed by the classification algorithms described herein. In some embodiments, these features may be directly included in each tree or algorithm of the ensemble, rather than merely being included in the collection of features from with a subset of features is randomly selected.

### 3. For plasma samples, use of the variant AF as more information to weigh the probabilities of each variant-containing gene or location:

Since the cell of origin is by definition clonal (it is the definition of the initial cell type from which DLBCL arose), variants that contribute to the cell of origin are more likely to be clonal variants than subclonal variants. In order to account for the likelihood of each variant to contribute to the COO information, the relative allele fractions (AF) of each variant detected can be incorporated into COO calling allowing heavier weighting of variants with higher AFs that are more likely to be clonal. This should allow separation of variants that truly represent cell of origin vs. the noise of low AF variants which only occurred well after tumorigenesis. Therefore, in some embodiments, the variant AF may be included in the collection of features analyzed by the classification algorithms. In some embodiments, these features may be directly included in each tree or algorithm of the ensemble, rather than merely being included in the collection of features from with a subset of features is randomly selected.

### Alternative embodiments

In some embodiments, instead of using an ensemble of XGBoost models for feature selection, other types of decision tree based models as described herein can be used for feature selection. Once the features are selected, an ensemble of XGBoost models or other decision tree based models can then be constructed. Feature selection can be performed as described above, or can be performed using another technique. For example, feature selection methods include supervised, unsupervised, and semi-supervised feature selection. See J. C. Ang, A. Mirzal, H. Haron and H. N. A. Hamed, "Supervised, Unsupervised, and Semi-Supervised Feature Selection: A Review on Gene Selection," in *IEEE*/*ACM Transactions on Computational Biology and Bioinformatics*, vol. 13, no. 5, pp. 971-989, 1 September 2016; Li, Jundong et al. "Feature Selection." ACM Computing Surveys 50.6 (2017): 1-45. Supervised feature selection involves using labeled data (i.e. labeled features), such as the identified genes and gene variants described above, in the feature selection process, while unsupervised feature selection involves unlabeled data and uses various structures and/or properties of the data, such as data variance, separability, and data distribution, in the feature selection process. Semi-supervised feature selection involves using a mix of labeled data with unlabeled data in the feature selection process.

In some embodiments, the data set can be separated into a training data set for feature evaluation and selection and constructing the model, and a validation data set for evaluating the performance and prediction accuracy of the models.

In some embodiments, features are classified and/or ranked according to their relevance or importance to the performance (i.e., prediction accuracy) of the model. In some embodiments, the features are also classified by their level of redundancy, by for example, identifying a level of correlation of one feature with one or more other features. In some embodiments, features that are highly correlated with another feature may be redundant and may be eliminated.

Feature evaluation can be performed using a variety of methods, such as the following: (1) filter or open-loop method that evaluate the feature characteristics based on dependency, information, distance, and consistency; (2) wrapper or closed looped method that uses error rate or performance accuracy to evaluate the features; (3) embedded method that integrates the feature selection algorithm into the machine learning algorithm; (4) hybrid model that utilizes two or more methods for feature evaluation, such as the filtering and wrapper methods; and (5) ensemble method that uses a plurality of models that each use a subsample of the data set for feature evaluation and selection, which are then combined and evaluated to form an aggregated set of selected features. Stopping criteria for determining when to stop the feature selection process can include (1) predetermined number of features, (2) predetermined number of iterations, (3) percentage of improvement over two consecutive iteration steps, and (4) use of an evaluation function (i.e. minimizing a cost or loss function).

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

## Claims

1. A computer-implemented method for cell of origin determination for a type of cancer, wherein the type of cancer is diffuse large B cell lymphoma, the method comprising: constructing a plurality of decision trees based on a collection of features, each decision tree comprising a random subset of features from the collection of features, wherein the features are genes that were identified based on one or more criteria from a plurality of blood or plasma samples from subjects having the type of cancer, wherein at least 10 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88, and wherein the criteria for identifying a gene comprises at least one of the following conditions:
(1) at least about 2% of the subjects from a first cell of origin class or a second cell of origin class have a variant in the gene;
(2) at most about 30% of all subjects with variants in the gene are unclassified; and
(3) a ratio of a first cell of origin class to a second cell of original class GCB or a ratio of the second cell of original class to the first cell of origin class is at least 55:45; and
training the plurality of decision trees based on the collection of features to create a cell of origin classifier
receiving a file comprising variant data from a patient, wherein the variant data was obtained from a blood or plasma sample from the patient; and
using the cell of origin classifier to determine a cell of origin based on the variant data in the file.

2. The method of claim 1, wherein at least 20 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

3. The method of claim 1, wherein at least 30 of the features from the collection of features is selected from the group of genes consisting of EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

4. The method of claim 1, wherein one of the features in the collection is variant location data.

5. The method of claim 4, wherein the variant location data comprises at least one of:
(1) a position of a variant in a gene;
(2) information about a domain region of a protein which is modified in a variant;
(3) information about a structural motif of a protein which is modified in a variant; and
(4) functional region of a protein which is modified in a variant.

6. The method of claim 1, wherein one of the features in the collection is variant allele fraction data.

## Patentansprüche

1. Computerimplementiertes Verfahren zur Bestimmung einer Ursprungszelle für einen Krebstyp, wobei der Krebstyp diffuses großzelliges B-Zell-Lymphom ist, wobei das Verfahren Folgendes umfasst:
Konstruieren einer Vielzahl von Entscheidungsbäumen basierend auf einer Sammlung von Merkmalen, wobei jeder Entscheidungsbaum eine zufällige Teilmenge von Merkmalen aus der Sammlung von Merkmalen umfasst, wobei die Merkmale Gene sind, die basierend auf einem oder mehreren Kriterien aus einer Vielzahl von Blut- oder Plasmaproben von Subjekten mit dem Krebstyp identifiziert wurden, wobei mindestens 10 der Merkmale aus der Sammlung von Merkmalen ausgewählt sind aus der Gruppe von Genen, bestehend aus EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88, und wobei die Kriterien zum Identifizieren eines Gens mindestens eine der folgenden Bedingungen umfassen:
(1) mindestens etwa 2 % der Subjekte aus einer ersten Ursprungszellklasse oder einer zweiten Ursprungszellklasse weisen eine Variante in dem Gen auf;
(2) höchstens etwa 30 % aller Subjekte mit Varianten in dem Gen sind nicht klassifiziert; und
(3) ein Verhältnis einer ersten Ursprungszellklasse zu einer zweiten Ursprungszellklasse GCB oder ein Verhältnis der zweiten Ursprungszellklasse zu der ersten Ursprungszellklasse beträgt mindestens 55:45; und
Trainieren der Vielzahl von Entscheidungsbäumen basierend auf der Sammlung von Merkmalen, um einen Ursprungszellen-Klassifikator zu erzeugen;
Empfangen einer Datei, die Variantendaten von einem Patienten umfasst, wobei die Variantendaten aus einer Blut- oder Plasmaprobe von dem Patienten erhalten wurden; und
Verwenden des Ursprungszellen-Klassifikators, um eine Ursprungszelle basierend auf den Variantendaten in der Datei zu bestimmen.

2. Verfahren nach Anspruch 1, wobei mindestens 20 der Merkmale aus der Sammlung von Merkmalen ausgewählt sind aus der Gruppe von Genen, bestehend aus EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

3. Verfahren nach Anspruch 1, wobei mindestens 30 der Merkmale aus der Sammlung von Merkmalen ausgewählt sind aus der Gruppe von Genen, bestehend aus EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

4. Verfahren nach Anspruch 1, wobei eines der Merkmale in der Sammlung Variantenortsdaten sind.

5. Verfahren nach Anspruch 4, wobei die Variantenortsdaten mindestens eines von Folgendem umfassen:
(1) eine Position einer Variante in einem Gen;
(2) Informationen über eine Domänenregion eines Proteins, die in einer Variante modifiziert ist;
(3) Informationen über ein Strukturmotiv eines Proteins, das in einer Variante modifiziert ist; und
(4) eine funktionelle Region eines Proteins, die in einer Variante modifiziert ist.

6. Verfahren nach Anspruch 1, wobei eines der Merkmale in der Sammlung Variantenallelfraktionsdaten sind.

## Revendications

1. Procédé mis en œuvre par ordinateur de détermination de cellule d'origine pour un type de cancer, dans lequel le type de cancer est le lymphome diffus à grandes cellules B, le procédé comprenant :
la construction d'une pluralité d'arbres de décision en se basant sur une collection de caractéristiques, chaque arbre de décision comprenant un sous-ensemble aléatoire de caractéristiques de la collection de caractéristiques, dans lequel les caractéristiques sont des gènes qui ont été identifiés en se basant sur un ou plusieurs critères d'une pluralité d'échantillons de sang ou de plasma provenant de sujets ayant le type de cancer, dans lequel au moins 10 des caractéristiques de la collection de caractéristiques sont choisies dans le groupe de gènes constitué par EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88, et dans lequel les critères d'identification d'un gène comprennent au moins l'une des conditions suivantes :
(1) au moins environ 2 % des sujets d'une première classe de cellule d'origine ou d'une seconde classe de cellule d'origine ont un variant dans le gène ;
(2) au plus environ 30 % de tous les sujets avec des variants dans le gène sont non classés ; et
(3) un rapport d'une première classe de cellule d'origine à une seconde classe de cellule d'origine GCB ou un rapport de la seconde classe de cellule d'origine à la première classe de cellule d'origine est d'au moins 55:45 ; et
l'entraînement de la pluralité d'arbres de décision en se basant sur la collection de caractéristiques pour créer un classificateur de cellule d'origine
la réception d'un fichier comprenant des données de variant d'un patient, dans lequel les données de variant ont été obtenues à partir d'un échantillon de sang ou de plasma du patient ; et
l'utilisation du classificateur de cellule d'origine pour déterminer une cellule d'origine en se basant sur les données de variant dans le fichier.

2. Procédé selon la revendication 1, dans lequel au moins 20 des caractéristiques de la collection de caractéristiques sont choisies dans le groupe de gènes constitué par EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

3. Procédé selon la revendication 1, dans lequel au moins 30 des caractéristiques de la collection de caractéristiques sont choisies dans le groupe de gènes constitué par EZH2, SGK1, GNAI3, IRF8, TNFRSF14, STAT6, BCL7A, KMT2D, SOCS1, RHOA, BCL2, STAT3, POU2F2, CD83, NFKBIA, CREBBP, CD58, TET2, KLHL6, CARD11, BCL6, MYC, PAX5, ZNF608, DUSP2, FOXO1, EP300, CCND3, ETS1, TMEM30A, PRDM1, IRF4, KLHL14, PIM1, IGLL5, CDKN2A, TBL1XR1, ZEB2, CD79B, MYD88.

4. Procédé selon la revendication 1, dans lequel l'une des caractéristiques dans la collection est des données de localisation de variant.

5. Procédé selon la revendication 4, dans lequel les données de localisation de variant comprennent au moins une parmi :
(1) une position d'un variant dans un gène ;
(2) des informations sur une région de domaine d'une protéine qui est modifiée dans un variant ;
(3) des informations sur un motif structurel d'une protéine qui est modifiée dans un variant ; et
(4) une région fonctionnelle d'une protéine qui est modifiée dans un variant.

6. Procédé selon la revendication 1, dans lequel l'une des caractéristiques dans la collection est des données de fraction d'allèle variant.
